(19) Europäisches Patentamt / European Patent Office / Office européen des brevets

(11) **EP 1 587 797 B1**

(12) **EUROPÄISCHE PATENTSCHRIFT**

(45) Veröffentlichungstag und Bekanntmachung des Hinweises auf die Patenterteilung:
**11.08.2010 Patentblatt 2010/32**

(21) Anmeldenummer: **04702691.9**

(22) Anmeldetag: **16.01.2004**

(51) Int Cl.:
***C07D 251/60*** *(2006.01)*

(86) Internationale Anmeldenummer:
**PCT/EP2004/000327**

(87) Internationale Veröffentlichungsnummer:
**WO 2004/065371 (05.08.2004 Gazette 2004/32)**

(54) **ZWEISTUFIGER REAKTOR FÜR DIE MELAMINSYNTHESE**

TWO-STAGE REACTOR FOR THE PRODUCTION OF MELAMINE

REACTEUR A DEUX ETAGES POUR LA SYNTHESE DE MELAMINE

(84) Benannte Vertragsstaaten:
**AT BE BG CH CY CZ DE DK EE ES FI FR GB GR HU IE IT LI LU MC NL PT RO SE SI SK TR**

(30) Priorität: **17.01.2003 DE 10301703**
**14.08.2003 DE 10337501**

(43) Veröffentlichungstag der Anmeldung:
**26.10.2005 Patentblatt 2005/43**

(73) Patentinhaber: **BASF SE**
**67056 Ludwigshafen (DE)**

(72) Erfinder:
- **KUHRS, Christian**
  **69115 Heidelberg (DE)**
- **DANZ, Eckehard**
  **67071 Ludwigshafen (DE)**
- **STEINER, Wolfgang**
  **67159 Friedelsheim (DE)**
- **RAHN, Ralf-Thomas**
  **68167 Mannheim (DE)**
- **GRASSLER, Thomas**
  **67117 Limburgerhof (DE)**
- **GEIER, Reiner**
  **68199 Mannheim (DE)**
- **HARTH, Klaus**
  **67317 Altleiningen (DE)**
- **HÖLZLE, Markus**
  **67281 Kirchheim (DE)**

(56) Entgegenhaltungen:
**DE-B- 1 209 570 US-A- 5 350 849**

- **DATABASE WPI Section Ch, Week 199614 Derwent Publications Ltd., London, GB; Class A41, AN 1996-136281 XP002276788 & JP 08 027126 A (MITSUI TOATSU CHEM INC), 30. Januar 1996 (1996-01-30) in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Anmeldung bezieht sich auf ein Verfahren zur Herstellung von Melamin durch katalytische Zersetzung von Harnstoff. Bei dem erfindungsgemäßen Verfahren handelt es sich um ein zweistufiges Verfahren, bei dem in den beiden Stufen jeweils Katalysatoren unterschiedlicher Acidität eingesetzt werden.

Melamin, dessen Struktur in der nachfolgenden Formel I wiedergegeben ist,

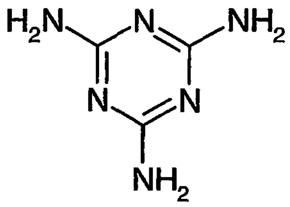

I

findet Verwendung zur Herstellung von Melamin-Harzen durch Umsetzung mit carbonylhaltigen Verbindungen. Die Harze werden u.a. als Kunststoffe sowie in Farben und Lacken eingesetzt. Die Herstellung von Melamin durch Zersetzung von Harnstoff ist eine bekannte Reaktion, die von der chemischen Industrie in mehreren Varianten benutzt wird. Prinzipiell wird zwischen dem Hochdruck- und dem Niederdruckverfahren unterschieden. Das Hochdruckverfahren wird bei Drükken von > ca. 80 bar (abs.) und Temperaturen > 370°C durchgerührt, wobei die Melaminsynthese auf nicht katalytische Weise erfolgt.

In Zusammenhang mit der vorliegenden Erfindung besitzt jedoch das Niederdruckverfahren, das bei Drücken von ca. 1 bis 10 bar (abs.) und Temperaturen von 370 bis 430°C durchgeführt wird, größere Bedeutung. Es ist bekannt, dass die Reaktion dabei in zwei Schritten abläuft. Im ersten, endothermen Schritt reagiert Harnstoff zu Ammoniak und Isocyansäure, die im zweiten, exothermen Schritt zu Melamin unter Freisetzung von $CO_2$ trimerisiert. Die nachfolgenden Gleichungen geben die einzelnen Umsetzungen wieder.

$$6\ H_2N\text{-}C(O)\text{-}NH_2 \rightarrow 6\ HN{=}C{=}O + 6\ NH_3 \quad \square H = 984\ kJ/mol$$

$$6\ HN{=}C{=}O \rightarrow C_3N_3(NH_2)_3 + 3\ CO_2 \quad \square H = -355\ kJ/mol$$

$$6\ H_2N\text{-}C(O)\text{-}NH_2 \rightarrow C_3N_3(NH_2)_3 + 6\ NH_3 + 3\ CO_2 \quad \square H = 629\ kJ/mol$$

Es existieren hauptsächlich drei Varianten des Niederdruckverfahrens, auf die nachfolgend näher eingegangen wird.

Bei dem Verfahren der Linz-Chemie wird die Umsetzung in zwei Stufen durchgeführt. In der ersten Stufe wird geschmolzener Harnstoff in einer Sand-Wirbelschicht zu Ammoniak und Isocyansäure zersetzt, bei 350°C und 3,5 bar (abs.) Anschließend wird in einem Festbettreaktor Isocyansäure bei 450°C und Atmosphärendruck katalytisch zu Melamin umgesetzt. Der Katalysator ist dabei generell ein Aluminiumoxid-Katalysator.

Das DSM-Stamicarbon-Verfahren ist ein einstufiges Verfahren, das bei ca. 7 bar (abs.) durchgeführt wird. Als Katalysator dienen Aluminiumsilicate, die als Wirbelschicht eingesetzt werden. Als Wirbelgas dient reiner Ammoniak, der über eine Abgasaufarbeitung rückgewonnen wird.

Schließlich existiert das BASF-Verfahren. Auch hier wird in der Wirbelschicht gearbeitet, als Katalysator dienen Aluminiumoxid- oder Aluminiumoxid-/Siliciumdioxid-Katalysatoren, die bei niedrigem Druck (ca. 2 bar abs.) betrieben werden. Als Gas für die Wirbelschicht dient dem Reaktor entstammendes Kreisgas enthaltend $NH_3$ und $CO_2$, das zuvor von Verunreinigungen befreit wurde, generell durch Behandeln mit einer Harnstoff schmelze, die Verunreinigungen aufnimmt.

Ein generell auftretendes Problem bei der Durchführung sämtlicher oben genannter katalytischer Verfahren - die prinzipiell gegenüber den nicht-katalytischen Verfahren den Vorteil einer einfacheren, kostengünstigeren apparativen Durchführung bieten - liegt in der Abscheidung höherer Kondensationsprodukte des Melamins auf der Oberfläche des Katalysators (Belag). Beispielsweise ist hier das sogenannte Melem ($C_6H_6N_{10}$, 2,5,8-Triamino-1,3,4,6,7,9,9b-Heptaazaphenalen) zu nennen, bei dem es sich um eine Dreikernverbindung aus drei annellierten Triazinringen handelt. Mit der übermäßigen Ablagerung der Kondensationsprodukte geht eine Desaktivierung des Katalysators einher, die dessen

Regenerierung beispielsweise durch thermische Behandlung und/oder Behandlung mit Wasserdampf, Luft oder Ammoniak erfordert, im Extremfall aber auch einen Austausch des Katalysators notwendig macht. Da sich zudem die Ablagerung der Kondensationsprodukte auf dem Katalysator relativ schnell vollzieht und diese eine Stationärkonzentration erreicht, tritt die Desaktivierung häufig bereits nach einem sehr kurzen Zeitraum ein, so dass ein periodisches Regenerieren wegen der kurzen Zeitabstände nicht in Frage kommt.

Über die notwendigen Eigenschaften bzw. Zusammensetzungen, die ein bei der MelaminSynthese eingesetzter Katalysator zum Erreichen einer hohen Ausbeute bzw. einer geringen Zersetzungsrate aufweisen muss, existieren bislang keine systematischen Untersuchungen bzw. Erkenntnisse.

In der JP-A 08 027 126 wird ein $\gamma$-$Al_2O_3$-Katalysator mit definierten Aciditätsgrenzen für die Melaminsynthese beansprucht.

In der Thianranqi Huagong, 2001, Band 26, Seiten 23 bis 25 (zitiert nach CA 136:135396) wird offenbart, dass aktive Katalysatoren für die Melaminsynthese durch Mischen von $Al_2O_3$ mit Zeolithen oder Zeolithen mit Metallkationen erhalten werden können. Die erhaltene Aktivität wird den aciden Zentren des Katalysators zugeschrieben.

DE 12 09 570 B offenbart ein Verfahren zur katalytischen Herstellung von Melamin durch Zersetzung von Harnstoff an Festbettkatalysatoren. Die thermische Zersetzung des Harnstoffs wird dabei in zwei Stufen in Gegenwart von Aluminiumoxidkatalysatoren vorgenommen.

US 5,350,849 A offenbart ein Verfahren zur katalytischen Herstellung von Melamin durch Zersetzung von Harnstoff an Feststoff-Katalysatoren. Dabei werden zwei Reaktoren verwendet, die beide $SiO_2$-$Al_2O_3$ als Katalysator enthalten.

Es hat sich jedoch gezeigt, dass durch den Einsatz von Katalysatoren mit erhöhter Acidität die Probleme der Katalysatordesaktivierung, insbesondere durch Belagbildung, und die damit verbundenen niedrigen Umsätze nicht gelöst werden konnten.

Die Aufgabe der vorliegenden Erfindung besteht darin, ein Verfahren bereitzustellen, mit dem hohe Umsätze und Melamin-Ausbeuten erreichbar sind, ohne dass eine frühzeitige Katalysatordesaktivierung durch Belagbildung eintritt, insbesondere unter den gewählten Reaktionsbedingungen.

Diese Aufgabe wird gelöst durch ein Verfahren zur katalytischen Herstellung von Melamin durch Zersetzung von Harnstoff an Feststoff-Katalysatoren unter Verwendung eines Haupt- und Nachreaktors, **dadurch gekennzeichnet, dass** im Hauptreaktor ein Katalysator geringer Lewis-Acidität und im Nachreaktor ein Katalysator höherer Lewis-Acidität eingesetzt wird.

Der vorliegenden Erfindung liegt die Erkenntnis zugrunde, dass der Einsatz eines Katalysators mit hoher Lewis-Acidität zwar einen hohen Umsatz des Edukts Harnstoff zu Melamin bewirkt und damit zu hohen Reaktionsausbeuten führt, jedoch auch schnell die Bildung von Ablagerungen auf dem eingesetzten Katalysator eintritt. Der erwünschte Effekt des hohen Umsatzes wird also schnell durch die Desaktivierung des Katalysators als Folge der Belagsbildung negativ kompensiert.

Da die Maßnahmen, die zum Vermeiden bzw. Rückgängigmachen von Ablagerungen getroffen wurden, kostspielig sind und außerdem die Belagbildung rasch eintritt, erweist es sich als günstig, die Melaminbildung in zwei getrennten Reaktoren (Haupt- und Nachreaktor) durchzuführen. Im Hauptreaktor wird ein Katalysator geringer Lewis-Acidität benutzt, wodurch ein vergleichsweise geringer Umsatz resultiert, jedoch auch nur eine geringe Belagbildung auftritt. Im Nachreaktor kommt ein Katalysator zum Einsatz, der eine höhere Lewis-Acidität aufweist. Vorzugsweise weist der Katalysator im Nachreaktor eine höhere Lewis-Acidität auf, wodurch ein sehr hoher Umsatz ermöglicht wird. Somit lässt sich ein hoher Gesamtumsatz erreichen, bei gleichzeitig geringer Desaktivierung sowohl des im Hauptreaktor als auch des im Nachreaktor eingesetzten Katalysators.

Im Hauptreaktor, in dem bereits sowohl die Spaltung des Harnstoffs zu Isocyansäure als auch die Trimerisierung zu Melamin ablaufen, insbesondere die letzte Reaktion jedoch nur unvollständig, kann der Katalysator prinzipiell in allen dem Fachmann bekannten Formen vorliegen, beispielsweise als Festbett, Wirbelschicht, zirkulierende Wirbelschicht oder Wanderbett. Vorzugsweise wird der Katalysator als Wirbelschicht eingesetzt.

Der im Hauptreaktor eingesetzte Katalysator enthält vorzugsweise mindestens ein Mineral aus der Gruppe der Aluminiumoxide, Siliciumoxide und Alumosilikate oder Mischungen verschiedener Aluminiumoxide, Siliciumoxide und/ oder Alumosilikate. Besonders bevorzugt enthält er mindestens ein Mineral aus der Gruppe Bayerit, Boehmit, Gibbsit, Montmorillonit, Bentonit und Muscovit, insbesondere Bentonit. Der Katalysator kann auch ganz aus den genannten Mineralien bestehen.

Die genannten Mineralien können zum Erzielen einer gewünschten Acidität gegebenenfalls vor dem Einsatz auf dem Fachmann bekannte Weise aktiviert worden sein, beispielsweise durch thermische Behandlung. Da im Allgemeinen bei einer thermischen Behandlung die Acidität der genannten Mineralien steigt, wird diese bei den im Hauptreaktor eingesetzten Katalysatoren generell nicht durchgeführt.

Die im Hauptreaktor eingesetzten Katalysatoren weisen vorzugsweise eine Oberflächen Lewis-Acidität von 0,3 bis 1,8, mehr bevorzugt 0,5 bis 1,5, insbesondere 0,8 bis 1,2 $\mu$mol/g auf. Die angegebenen Werte wurden durch Aciditätsmessungen in einer Hochvakuum-Fourier-Transform-Infrarot-Anlage (HV-FTIR) bei einer Temperatur von 390

°C mit Pyridin als Sondenmolekül durchgeführt und die durch unterschiedliche IR-Absorptionsbanden charakterisierte Lewiszentren quantitativ durch Integration der Peakflächen erfasst. Es wurde dabei die Methode angewandt, die in Turk. J. Chem. 23 (1999), Seite 319 bis 327 beschrieben ist. Die Werte gelten für einen Innendurchmesser der Presslinghalter von 5,1 mm. Typische Wirbelschichtkatalysatoren weisen BET-Oberflächen von 50 bis 350 $m^2$/g, bevorzugt von 100 bis 250 $m^2$/g, auf. Porenvolumina liegen zwischen 0,1 und 1,0 ml/g. Die durchschnittliche Partikelgröße der Katalysatoren beträgt 10 bis 500 $\mu$m.

Das Verfahren in Anwesenheit der genannten Katalysatoren wird bei Temperaturen von 350 bis 450°C, vorzugsweise 380 bis 420°C, einem Absolutdruck von 1 bis 15 bar, vorzugsweise 1 bis 10 bar, insbesondere 5 bis 8 bar, einer Verweilzeit über das Wirbelbett von 1 bis 50 s, vorzugsweise 2 bis 30 s und einer Katalysatorbelastung von 20 bis 700 kg Harnstoff/t (Kat) • h, vorzugsweise 50 bis 500 kg Harnstoff/t (Kat) • h betrieben.

Der Hauptreaktor kann üblicherweise eine zylindrische oder konische Form aufweisen. In einer Ausführungsform der vorliegenden Erfindung ist der als Wirbelschichtreaktor vorliegende Hauptreaktor konisch ausgebildet. Dadurch wird eine höhere Geschwindigkeit des eintretenden Gases und somit ein stabileres Wirbelverhalten erreicht.

Der im Nachreaktor eingesetzte Katalysator weist vorzugsweise gegenüber dem im Hauptreaktor eingesetzten Katalysator eine 1,5- bis 6-fach, bevorzugt 3- bis 5-fach höhere volumennormierte Oberflächen-Lewis-Acidität unter Reaktionsbedingungen auf.

Die Oberflächen-Acidität der im Nachreaktor eingesetzten Katalysatoren liegt vorzugsweise bei Werten von 2 bis 12, mehr bevorzugt 3 bis 10, insbesondere 3,5 bis 6 $\mu$mol/g. Die angegebenen Werte wurden durch Aciditätsmessungen in einer Hochvakuum-Fourier-Transform-Infrarot-Anlage (HV-FTIR) bei einer Temperatur von 390 °C mit Pyridin als Sondenmolekül durchgeführt und die durch unterschiedliche IR-Absorptionsbanden charakterisierte Lewiszentren quantitativ durch Integration der Peakflächen erfaßt. Es wurde dabei die Methode angewandt, die in Turk. J. Chem. 23 (1999), Seite 319 bis 327 beschrieben ist. Die Werte gelten für einen Innendurchmesser der Presslinghalter von 5,1 mm.

Wie bei den im Hauptreaktor eingesetzten Katalysatoren enthält der Katalysator im Nachreaktor vorzugsweise mindestens ein Mineral aus der Gruppe der Aluminiumoxide, Siliciumoxide und Alumosilikate oder Mischungen von Aluminiumoxiden, Siliciumoxiden und/oder Alumosilikaten. Die im Nachreaktor eingesetzten Katalysatoren enthalten 0 bis 60 Gew.-%, vorzugsweise 5 bis 50 Gew.-%, $SiO_2$ und 100 bis 40 Gew.-%, vorzugsweise 95 bis 50 Gew.-%, $Al_2O_3$. Vorzugsweise werden Alumosilikat-Katalysatoren eingesetzt.

Die Katalysatoren weisen BET-Oberflächen von 150 bis 400 $m^2$/g, vorzugsweise 200 bis 350 $m^2$/g, auf.

Die zum Erreichen der notwendigen Acidität erforderlichen Maßnahmen sind dem Fachmann bekannt. Dies kann durch Einbau von Ionen unterschiedlicher Wertigkeit in ein gegebenes Mineral (beispielsweise Siliciumdioxid in Aluminiumoxid) und/oder Wärmebehandlung erfolgen. In einer bevorzugten Ausführungsform der vorliegenden Erfindung werden die genannten Mineralien vor dem Einsatz durch Temperaturbehandlung aktiviert, bei Temperaturen von 350 bis 950°C, vorzugsweise 450 bis 750°C.

Die Porenvolumina der Katalysatoren liegen bei 0,1 bis 1,5 ml/g, vorzugsweise bei 0,2 bis 0,9 ml/g ($N_2$) bzw. 0,1 bis 2,0 ml/g, vorzugsweise 0,2 bis 1,0 ml/g (Hg-Porosometrie). Die Porendurchmesser betragen 10 bis 100 Å, vorzugsweise 30 bis 90 Å.

Das erfindungsgemäße Verfahren wird im Nachreaktor bei Verweilzeiten von 0,1 bis 20 s, vorzugsweise 0,5 bis 10 s, und Katalysatorbelastungen von 0,05 bis 2 g HNCO/g (Kat) • h, vorzugsweise 0,1 bis 1 g HNCO/g (Kat) • h durchgeführt. Die Temperatur beträgt 350 bis-500 °C, bevorzugt 390 bis 450 °C, der Druck liegt bei Werten von 1 bis 15 bar absolut, vorzugsweise 1 bis 10 bar, insbesondere 5 bis 8 bar absolut.

Im Nachreaktor kann der Katalysator in einer geeigneten, dem Fachmann bekannten Form vorliegen, also beispielsweise als Festbett oder Wirbelbett. Es hat sich als vorteilhaft erwiesen, wenn der Katalysator im Nachreaktor in einer Form vorliegt, bei der während der Reaktion nur eine geringe Rückvermischung eintritt. Dies ist beispielsweise bei Festbettkatalysatoren der Fall, so dass der Einsatz eines Festbettkatalysators im Nachreaktor bevorzugt ist. Vorteilhafterweise liegt der Festbettkatalysator als Formkörper vor. Vorzugsweise werden dabei Formkörper gewählt, die durchgängig für den aus dem Hauptreaktor herausgetragenen Katalysatorfeinstaub sind, beispielsweise Hohlstränge, Monolithe, Sternstränge, Tabletten oder Splitt. Besonders geeignet sind Wabenkörper oder Hohlstränge, insbesondere Wabenkörper. Wabenkörper weisen von den genannten Formkörpern die besten Eigenschaften hinsichtlich der Druckdifferenz beim Durchgang der Reaktionsgase auf.

Vorzugsweise werden Wabenkörper eingesetzt, die vollständig aus $\gamma$-Aluminiumoxid bestehen oder im Wesentlichen aus $\gamma$-Aluminiumoxid bestehen. Bevorzugt sind Wabenkörper, die 60 bis 100 Gew.-% $\gamma$-$Al_2O_3$ und 0 bis 40 Gew.-% $SiO_2$ enthalten.

Die zu Wabenkörpern formbare Masse wird trocken gemischt und mit einem Peptisierungsmittel, vorzugsweise Salpetersäure, und mit Wasser versetzt und danach gekollert. Geeignete Peptisierungsmittel sind dem Fachmann bekannt. Gegebenenfalls können weiterhin organische Hilfsmittel zugesetzt werden, die sich in der Hitze rückstandsfrei zersetzen. Beispiele sind Carbonate und Cellulosederivate. Konkrete Beispiele umfassen Ammoniumcarbonat, Ammoniumoxalat und Hydroxymethylcellulose (etwa die unter dem Namen Walocel®, Wolff Walsrode vertriebene). Die bewegte Masse wird dann unter Druck zur gewünschten Wabenkörpergeometrie extrudiert. Die Formkörper werden getrocknet

und abschließend kalziniert, vorzugsweise bei einer Temperatur < 600°C.

Vorzugsweise wird das Verfahren so durchgeführt, dass im Hauptreaktor der größte Teil des Umsatzes und im Nachreaktor ein kleinerer Teil des Umsatzes (Restumsatz) erfolgt.

Die Erfindung wird nun in den nachfolgenden Beispielen näher erläutert. Bei der Bezugnahme auf die Zeichnungen bedeuten: A = Umsatz [%]; B = organischer Belag [Gew.-%]; C = Laufzeit [h].

**BEISPIELE**

**Beispiel 1 (Vergleichsbeispiel)**

In einem Pilotreaktor mit einem Durchmesser von 80 cm und einer Katalysatorschütthöhe von ca. 8 m wurde bei einer Temperatur von ca. 400°C Harnstoff zu Melamin umgesetzt. Die drei untersuchten Katalysatoren (kalziniertes siliciumdotiertes Aluminiumoxid (Kat 1), kalziniertes Aluminiumoxid (Kat 2) sowie nichtkalziniertes Alumosilicat vom Montmorillonit-Typ (Kat 3)) hatten Lewis-Aciditäten unter Reaktionsbedingungen von 4,4 beziehungsweise 3,6 und 1,0 μmol/g. Die Wirbelgasmenge betrug ca. 300 $Nm^3/h$.

Wie Abb. 1 zeigt, liegt der Anfangsumsatz des am stärksten aciden Katalysators (Kat 1) mit ca. 90% am höchsten. Eine Katalysatordesaktivierung findet jedoch bereits nach ca. 250 Stunden Laufzeit statt, nach 450 Stunden liegt der Umsatz bereits unter 60%. Einhergehend mit der Katalysatordesaktivierung ist der Aufbau von organischem Belag auf dem Katalysator, der für die Desaktivierung verantwortlich ist.

Der mit einer Lewis-Acidität von 3,6 μmol/g etwas weniger acide Katalysator (Kat 2) zeigt einen etwas niedrigeren Anfangsumsatz von ca. 85%, der auch hier parallel mit der Bildung organischen Belags abnimmt (Abb. 2).

Abbildung 3 zeigt den entsprechenden Test mit dem am wenigsten aciden Katalysator (Kat 3, Lewis-Acidität lediglich 1 μmol/g). Der Katalysator zeigt einen Umsatz von lediglich ca. 75%, läuft jedoch aufgrund des konstanten organischen Belages mit konstantem Umsatz.

Es zeigt sich somit, dass acide Katalysatoren zwar einen hohen Umsatz gewährleisten, jedoch schnell desaktivieren. Katalysatoren mit geringer Acidität sind weniger aktiv, desaktivieren aber nur unwesentlich.

**Beispiel 2**

In einen Festbett-Nachreaktor eines Durchmessers von 13,5 cm und einer Katalysatorschütthöhe von 1,5 m wurden 30 $Nm^3/h$ eines aus einem Wirbelschichtreaktor stammenden Gases geleitet, der mit dem am wenigsten aciden Katalysator (Kat 3) aus Beispiel 1 betrieben wurde.

Bei dem im Festbett-Nachreaktor eingesetzten Katalysator handelte es sich um 10x20x5 mm Hohlstränge aus siliciumdotiertem Aluminiumoxid der Zusammensetzung 95% $Al_2O_3$ und 5% $SiO_2$, die nach Verstrangung über Nacht bei 550 °C kalziniert wurden.

Bei einer Temperatur von ca. 400 °C und einem Druck von 1,5 bar absolut konnte demonstriert werden, dass eine Standzeit > 1500 Stunden bei gleichzeitig > 90% Gesamtumsatz ohne Desaktivierung der Katalysatoren möglich ist (Abb. 4).

Durch die Kombination von Haupt- und Nachreaktor mit Katalysatoren unterschiedlicher Acidität wird folglich ein hoher Umsatz bei hoher Selektivität, verbunden mit geringer Katalysatordesaktivierung beobachtet.

In der nachfolgenden Tabelle sind die Anfangs- und Endwerte der jeweiligen Umsätze der Beispiele 1 und 2 noch einmal aufgeführt.

**Tabelle 1: Umsätze der Beispiele 1 und 2**

| | $Umsatz_{Anfang}$(%) | $Umsatz_{500\ h}$ (%) |
|---|---|---|
| Katalysator 1 | 88 | 56 |
| Katalysator 2 | 83 | 51 |
| Katalysator 3 | 73; konstant | |
| Katalysator 3 plus Nachreaktor | 92; konstant | |

**Beispiel 3**

3 kg aus 5% $SiO_2$ und 95% Aluminiumoxid-hydroxid bestehenden Materials und 7 kg eines aus 5% $SiO_2$ und 95% $\gamma$-$Al_2O_3$ bestehenden Materials werden 5 Minuten trocken gemischt und nach Zugabe von 0,635 kg 69,3%iger $HNO_3$ mit 2,5 kg VE-Wasser verdünnt und beim Kollern in einem Mix-Muller nochmals mit 4,3 kg VE-Wasser versetzt.

Bei einem Formdruck von 50 bar und einer Temperatur von 20 °C werden Wabenkörper mit einer Kantenlänge von 45 x 45 mm und einer Länge von 320 mm extrudiert, die 6 x 6 Zellen mit einem Innenmaß von 5,7 x 5,7 mm und einer Stegdicke von 1,8 mm besitzen. Die Formkörper werden bei Raumtemperatur getrocknet. Im Trockenschrank werden die Waben 24 Stunden bei 30 °C, dann in 10 °C-Schritten jeweils 24 Stunden bis 60 °C getrocknet. Die Waben werden weitere 24 Stunden bei 60 °C getrocknet. Abschließend werden die Waben bei 500 °C sieben Stunden kalziniert.

**Patentansprüche**

1. Verfahren zur katalytischen Herstellung von Melamin durch Zersetzung von Harnstoff an Feststoff-Katalysatoren unter Verwendung eines Haupt- und Nachreaktors, **dadurch gekennzeichnet, dass** im Hauptreaktor ein Katalysator geringer Lewis-Acidität und im Nachreaktor ein Katalysator höherer Lewis-Acidität eingesetzt wird.

2. Verfahren nach Anspruch 1, **dadurch gekennzeichnet, dass** der Katalysator im Hauptreaktor mindestens ein Mineral aus der Gruppe bestehend aus Aluminiumoxiden, Siliciumoxiden und Alumosilikaten oder Mischungen davon enthält, vorzugsweise mindestens ein Mineral aus der Gruppe Bayerit, Boehmit, Gibbsit, Montmorillonit, Bentonit und Muscovit, insbesondere Bentonit.

3. Verfahren nach Anspruch 1 oder 2, **dadurch gekennzeichnet, dass** der Katalysator im Hauptreaktor als Wirbelschicht vorliegt.

4. Verfahren nach Anspruch 3, **dadurch gekennzeichnet, dass** der Hauptreaktor konisch oder zylindrisch, vorzugsweise konisch, ausgebildet ist.

5. Verfahren nach einem der Ansprüche 1 bis 4, **dadurch gekennzeichnet, dass** die Lewis-Aciditat des Katalysators im Nachreaktor eine 1,5-6 fach, bevorzugt 3-5 fach höhere volumennormierte Oberflächen-Lewis-Acidität unter Reaktionsbedingungen aufweist.

6. Verfahren nach einem der Ansprüche 1 bis 5, **dadurch gekennzeichnet, dass** die Acidität des im Hauptreaktor eingesetzten Katalysators bei Werten von 0,3 bis 1,8, vorzugsweise 0,5 bis 1,5, insbesondere 0,8 bis 1,2 μmol/g liegt.

7. Verfahren nach einem der Ansprüche 1 bis 6, **dadurch gekennzeichnet, dass** die Acidität des im Nachreaktor eingesetzten Katalysators bei Werten von 2 bis 12, vorzugsweise 3 bis 10, insbesondere 3,5 bis 6 μmol/g liegt.

8. Verfahren nach einem der Ansprüche 1 bis 7, **dadurch gekennzeichnet, dass** der Katalysator im Nachreaktor 0 bis 60 Gew.-%, vorzugsweise 5 bis 50 Gew.-%, $SiO_2$ und 100 bis 40 Gew.-%, vorzugsweise 95 bis 50 Gew.-%, $Al_2O_3$ enthält.

9. Verfahren nach einem der Ansprüche 1 bis 8, **dadurch gekennzeichnet, dass** der Katalysator im Nachreaktor mindestens ein Mineral aus der Gruppe der Aluminiumoxide, Siliciumoxide und Alumosilikate oder Mischungen von Aluminiumoxiden, Siliciumoxiden und/oder Alumosilikaten enthält, vorzugsweise Alumosilikat.

10. Verfahren nach einem der Ansprüche 1 bis 9, **dadurch gekennzeichnet, dass** der Katalysator im Nachreaktor vor dem Einsatz aktiviert wird, bei Temperaturen von 350 bis 950 °C, vorzugsweise 450 bis 750 °C.

11. Verfahren nach einem der Ansprüche 1 bis 10, **dadurch gekennzeichnet, dass** der Katalysator im Nachreaktor eine BET-Oberfläche von 150 bis 400 $m^2$/g, vorzugsweise 200 bis 350 $m^2$/g, aufweist.

12. Verfahren nach einem der Ansprüche 1 bis 11, **dadurch gekennzeichnet, dass** die Porenvolumina der Katalysatoren bei 0,1 bis 1,5 ml/g, vorzugsweise bei 0,2 bis 0,9 ml/g ($N_2$) bzw. 0,1 bis 2,0 ml/g, vorzugsweise 0,2 bis 1,0 ml/g (Hg-Porosometrie) liegen und die Porendurchmesser 10 bis 100 Å, vorzugsweise 30 bis 90 Å, betragen.

13. Verfahren nach einem der Ansprüche 1 bis 12, **dadurch gekennzeichnet, dass** die Verweilzeiten im Nachreaktor bei Werten von 0,1 bis 20 s, vorzugsweise 0,5 bis 10 s, und die Katalysatorbelastungen bei Werten von 0,05 bis 2 g HNCO/g (Kat) • h, vorzugsweise 0,1 bis 1 g HNCO/g (Kat) • h, liegen.

14. Verfahren nach einem der Ansprüche 1 bis 13, **dadurch gekennzeichnet, dass** die Verweilzeiten im Hauptreaktor bei Werten von 1 bis 50 s, vorzugsweise 2 bis 30 s, und die Katalysatorbelastungen bei Werten von 20 bis 700,

vorzugsweise 50 bis 500 kg Harnstoff/t (Kat) • h liegen.

15. Verfahren nach einem der Ansprüche 1 bis 14, **dadurch gekennzeichnet, dass** die Umsetzung im Hauptreaktor bei einer Temperatur von 350 bis 450°C, vorzugsweise 380 bis 420°C, und einem Druck von 1 bis 15 bar, vorzugsweise 1 bis 10, insbesondere 5 bis 8 bar absolut, durchgeführt wird.

16. Verfahren nach einem der Ansprüche 1 bis 15, **dadurch gekennzeichnet, dass** die Umsetzung im Nachreaktor bei einer Temperatur von 350 bis 500 °C, bevorzugt 390 bis 450 °C, und einem Druck von 1 bis 15 bar, vorzugsweise 1 bis 10 bar, insbesondere 5 bis 8 bar absolut, durchgeführt wird.

17. Verfahren nach einem der Ansprüche 1 bis 16, **dadurch gekennzeichnet, dass** der Katalysator im Nachreaktor als Festbett, vorzugsweise als Formkörper, mehr bevorzugt als Monolith, Hohlstrang, Sternstrang, Tablette oder Splitt, insbesondere als Wabenkörper vorliegt.

18. Verfahren nach einem der Ansprüche 1 bis 17, **dadurch gekennzeichnet, dass** der Katalysator im Hauptreaktor als Wirbelschicht und im Nachreaktor als Festbett vorliegt.

**Claims**

1. A process for the catalytic preparation of melamine by decomposition of urea over solid catalysts using a main reactor and an after-reactor, wherein a catalyst having a low Lewis acidity is used in the main reactor and a catalyst having a higher Lewis acidity is used in the after-reactor.

2. The process according to claim 1, wherein the catalyst in the main reactor comprises at least one mineral from the group consisting of aluminum oxides, silicon oxides and aluminosilicates and mixtures thereof, preferably at least one mineral from the group consisting of bayerite, boehmite, gibbsite, montmorillonite, bentonite and muscovite, in particular bentonite.

3. The process according to claim 1 or 2, wherein the catalyst in the main reactor is present as a fluidized bed.

4. The process according to claim 3, wherein the main reactor has a conical or cylindrical, preferably conical, configuration.

5. The process according to any of claims 1 to 4, wherein the Lewis acidity of the catalyst in the after-reactor has a volume-standardized surface Lewis acidity under reaction conditions which is higher by a factor of 1.5-6, preferably 3-5.

6. The process according to any of claims 1 to 5, wherein the acidity of the catalyst used in the main reactor is from 0.3 to 1.8 μmol/g, preferably from 0.5 to 1.5 μmol/g, in particular from 0.8 to 1.2 μmol/g.

7. The process according to any of claims 1 to 6, wherein the acidity of the catalyst used in the after-reactor is from 2 to 12 μmol/g, preferably from 3 to 10 μmol/g, in particular from 3.5 to 6 μmol/g.

8. The process according to any of claims 1 to 7, wherein the catalyst in the after-reactor comprises from 0 to 60% by weight, preferably from 5 to 50% by weight, of $SiO_2$ and from 100 to 40% by weight, preferably from 95 to 50% by weight, of $Al_2O_3$.

9. The process according to any of claims 1 to 8, wherein the catalyst in the after-reactor comprises at least one mineral from the group consisting of aluminum oxides, silicon oxides and aluminosilicates and mixtures of aluminum oxides, silicon oxides and/or aluminosilicates, preferably aluminosilicate.

10. The process according to any of claims 1 to 9, wherein the catalyst in the after-reactor is activated at from 350 to 950°C, preferably from 450 to 750°C, before use.

11. The process according to any of claims 1 to 10, wherein the catalyst in the after-reactor has a BET surface area of from 150 to 400 $m^2/g$, preferably from 200 to 350 $m^2/g$.

12. The process according to any of claims 1 to 11, wherein the pore volumes of the catalysts are from 0.1 to 1.5 ml/g, preferably from 0.2 to 0.9 ml/g ($N_2$), or from 0.1 to 2.0 ml/g, preferably from 0.2 to 1.0 ml/g (Hg porosimetry), and the pore diameters are from 10 to 100 Å, preferably from 30 to 90 Å.

13. The process according to any of claims 1 to 12, wherein the residence times in the after-reactor are from 0.1 to 20 s, preferably from 0.5 to 10 s, and the space velocities over the catalyst are from 0.05 to 2 g of HNCO/g (cat) • h, preferably from 0.1 to 1 g of HNCO/g (cat) • h.

14. The process according to any of claims 1 to 13, wherein the residence times in the main reactor are from 1 to 50 s, preferably from 2 to 30 s, and the space velocities over the catalyst are from 20 to 700 kg of urea/t (cat) • h, preferably from 50 to 500 kg of urea/t (cat) • h.

15. The process according to any of claims 1 to 14, wherein the reaction in the main reactor is carried out at from 350 to 450°C, preferably from 380 to 420°C, and a pressure of from 1 to 15 bar, preferably from 1 to 10 bar, in particular from 5 to 8 bar absolute.

16. The process according to any of claims 1 to 15, wherein the reaction in the after-reactor is carried out at from 350 to 500°C, preferably from 390 to 450°C, and a pressure of from 1 to 15 bar, preferably from 1 to 10 bar, in particular from 5 to 8 bar absolute.

17. The process according to any of claims 1 to 16, wherein the catalyst in the after-reactor is present as a fixed bed, preferably as shaped bodies, more preferably as monolith, hollow extrudate, star extrudate, pellets or crushed material, in particular as honeycomb.

18. The process according to any of claims 1 to 17, wherein the catalyst in the main reactor is present as a fluidized bed and that in the after-reactor is present as a fixed bed.

**Revendications**

1. Procédé pour la préparation catalytique de mélamine par la décomposition d'urée sur des catalyseurs solides en utilisant un réacteur principal et un post-réacteur, **caractérisé en ce qu'**on utilise dans le réacteur principal un catalyseur de faible acidité de Lewis et dans le post-réacteur un catalyseur d'acidité de Lewis supérieure.

2. Procédé selon la revendication 1, **caractérisé en ce que** le catalyseur dans le réacteur principal contient au moins un minéral du groupe constitué par les oxydes d'aluminium, les oxydes de silicium et les aluminosilicates ou leurs mélanges, de préférence au moins un minéral du groupe formé par la bayérite, la boehmite, la gibbsite, la montmorillonite, la bentonite et la muscovite, en particulier la bentonite.

3. Procédé selon la revendication 1 ou 2, **caractérisé en ce que** le catalyseur dans le réacteur principal se trouve sous forme de couche tourbillonnante.

4. Procédé selon la revendication 3, **caractérisé en ce que** le réacteur principal présente une réalisation conique ou cylindrique, de préférence conique.

5. Procédé selon l'une quelconque des revendications 1 à 4, **caractérisé en ce que** l'acidité de Lewis du catalyseur dans le post-réacteur présente une acidité de Lewis de surface normalisée par rapport au volume supérieure d'un facteur 1,5-6, de préférence supérieure d'un facteur 3-5 dans les conditions de réaction.

6. Procédé selon l'une quelconque des revendications 1 à 5, **caractérisé en ce que** l'acidité du catalyseur utilisé dans le réacteur principal se situe à des valeurs de 0,3 à 1,8, de préférence de 0,5 à 1,5, en particulier de 0,8 à 1,2 μmole/g.

7. Procédé selon l'une quelconque des revendications 1 à 6, **caractérisé en ce que** l'acidité du catalyseur utilisé dans le post-réacteur se situe à des valeurs de 2 à 12, de préférence de 3 à 10, en particulier de 3,5 à 6 μmoles/g.

8. Procédé selon l'une quelconque des revendications 1 à 7, **caractérisé en ce que** le catalyseur dans le post-réacteur contient 0 à 60% en poids, de préférence 5 à 50% en poids, de $SiO_2$ et 100 à 40% en poids, de préférence 95 à 50% en poids, d'$Al_2O_3$.

9. Procédé selon l'une quelconque des revendications 1 à 8, **caractérisé en ce que** le catalyseur dans le post-réacteur contient au moins un minéral du groupe formé par les oxydes d'aluminium, les oxydes de silicium et les aluminosilicates, ou des mélanges d'oxydes d'aluminium, d'oxydes de silicium et/ou d'aluminosilicates, de préférence l'aluminosilicate.

10. Procédé selon l'une quelconque des revendications 1 à 9, **caractérisé en ce que** le catalyseur dans le post-réacteur est activé avant l'utilisation, à des températures de 350 à 950°C, de préférence de 450 à 750°C.

11. Procédé selon l'une quelconque des revendications 1 à 10, **caractérisé en ce que** le catalyseur dans le post-réacteur présente une surface BET de 150 à 400 $m^2$/g, de préférence de 200 à 350 $m^2$/g.

12. Procédé selon l'une quelconque des revendications 1 à 11, **caractérisé en ce que** les volumes de pores des catalyseurs se situent à 0,1 jusqu'à 1,5 ml/g, de préférence à 0,2 jusqu'à 0,9 ml/g ($N_2$) ou à 0,1 jusqu'à 2,0 ml/g, de préférence à 0,2 jusqu'à 1,0 ml/g (porosimétrie par Hg) et les diamètres des pores sont de 10 à 100 Å, de préférence de 30 à 90 Å.

13. Procédé selon l'une quelconque des revendications 1 à 12, **caractérisé en ce que** les temps de séjour dans le post-réacteur se situent à des valeurs de 0,1 à 20 s, de préférence de 0,5 à 10 s, et les charges de catalyseur se situent à des valeurs de 0,05 à 2 g de HNCO/g (Cat) * h, de préférence de 0,1 à 1 g de HNCO/g (Cat) * h.

14. Procédé selon l'une quelconque des revendications 1 à 13, **caractérisé en ce que** les temps de séjour dans le réacteur principal se situent à des valeurs de 1 à 50 s, de préférence de 2 à 30 s, et les charges de catalyseur se situent à des valeurs de 20 à 700, de préférence de 50 à 500 kg d'urée/t (Cat) * h.

15. Procédé selon l'une quelconque des revendications 1 à 14, **caractérisé en ce que** la transformation dans le réacteur principal est réalisée à une température de 350 à 450°C, de préférence de 380 à 420°C, et à une pression de 1 à 15 bars, de préférence de 1 à 10 bars, en particulier de 5 à 8 bars abs.

16. Procédé selon l'une quelconque des revendications 1 à 15, **caractérisé en ce que** la transformation dans le post-réacteur est réalisée à une température de 350 à 500°C, de préférence de 390 à 450°C, et à une pression de 1 à 15 bars, de préférence de 1 à 10 bars, en particulier de 5 à 8 bars abs.

17. Procédé selon l'une quelconque des revendications 1 à 16, **caractérisé en ce que** le catalyseur se trouve dans le post-réacteur sous forme de lit fixe, de préférence sous forme de corps façonné, plus préférablement sous forme de monolithe, de brin creux, de brin en étoile, de comprimé ou de fragments, en particulier sous forme de corps en nid d'abeille.

18. Procédé selon l'une quelconque des revendications 1 à 17, **caractérisé en ce que** le catalyseur dans le réacteur principal se trouve sous forme de couche tourbillonnante et dans le post-réacteur sous forme de lit fixe.

FIG.1

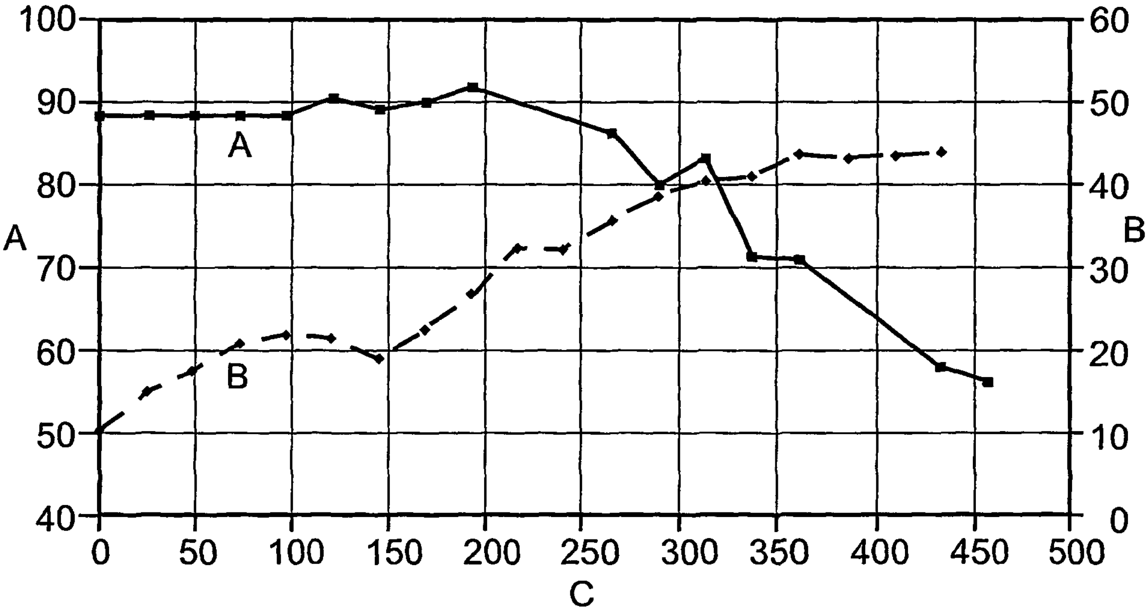
100
90
80
70
60
50
40
A
60
50
40
30
20
10
0
B
0
50
100
150
200
250
300
350
400
450
500
C
A
B

FIG.2

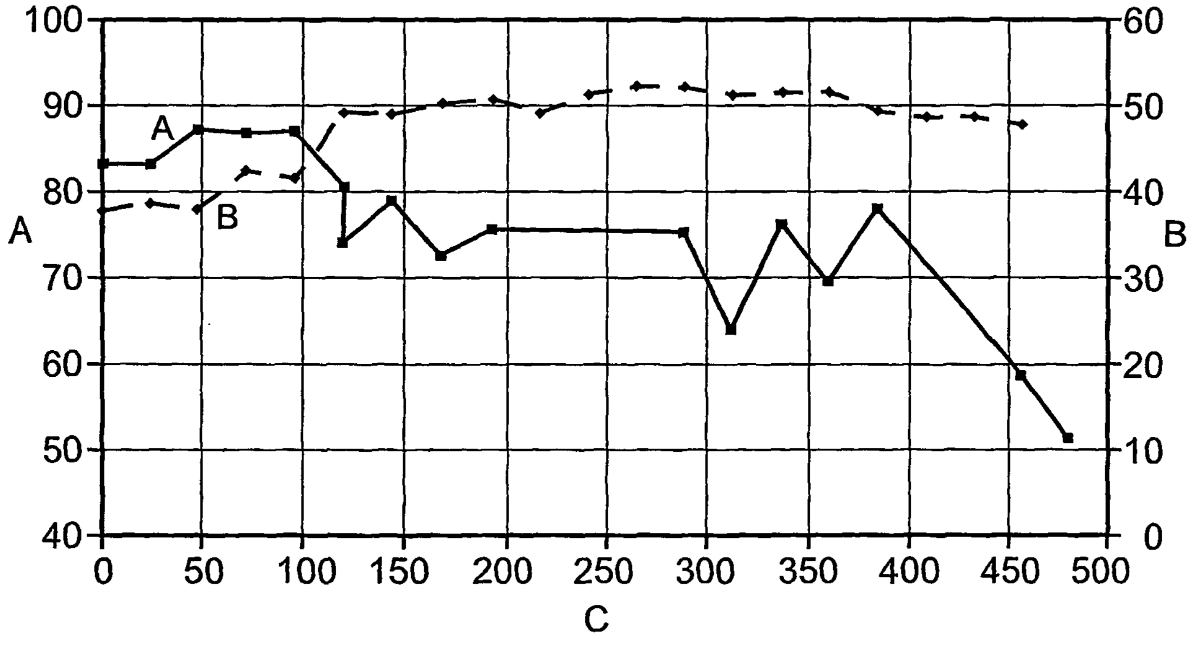
100
90
80
70
60
50
40
A
60
50
40
30
20
10
0
B
0
50
100
150
200
250
300
350
400
450
500
C
A
B

FIG.3

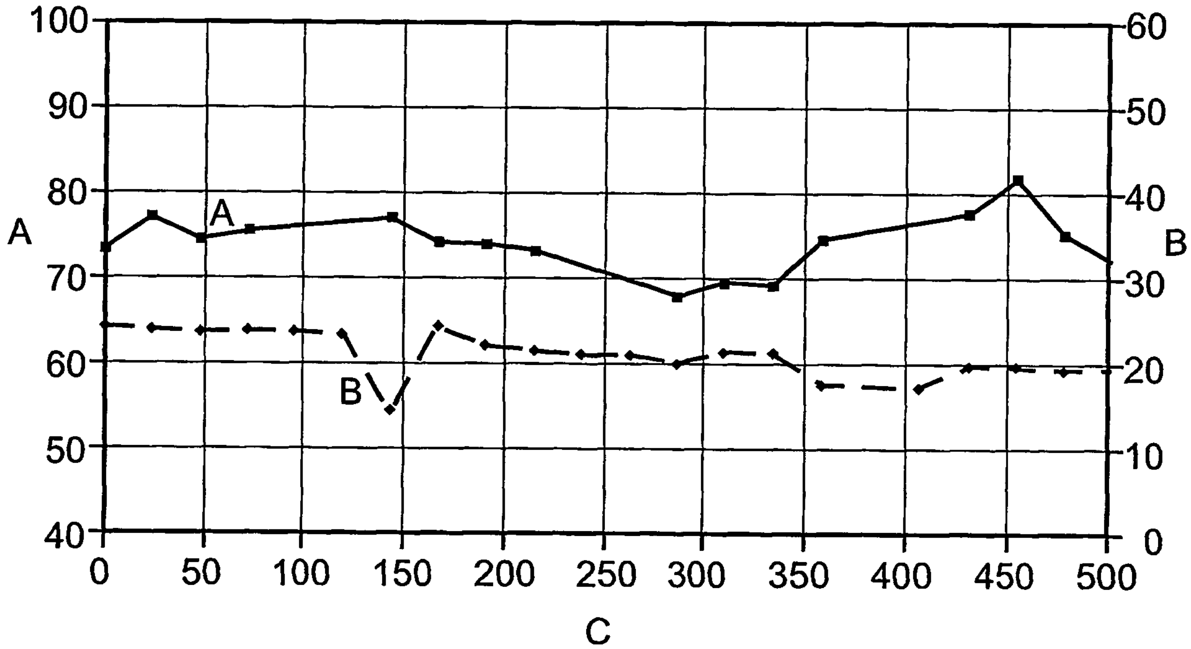
100
90
80
70
60
50
40
A
A
B
60
50
40
30
20
10
0
B
0
50
100
150
200
250
300
350
400
450
500
C

FIG.4

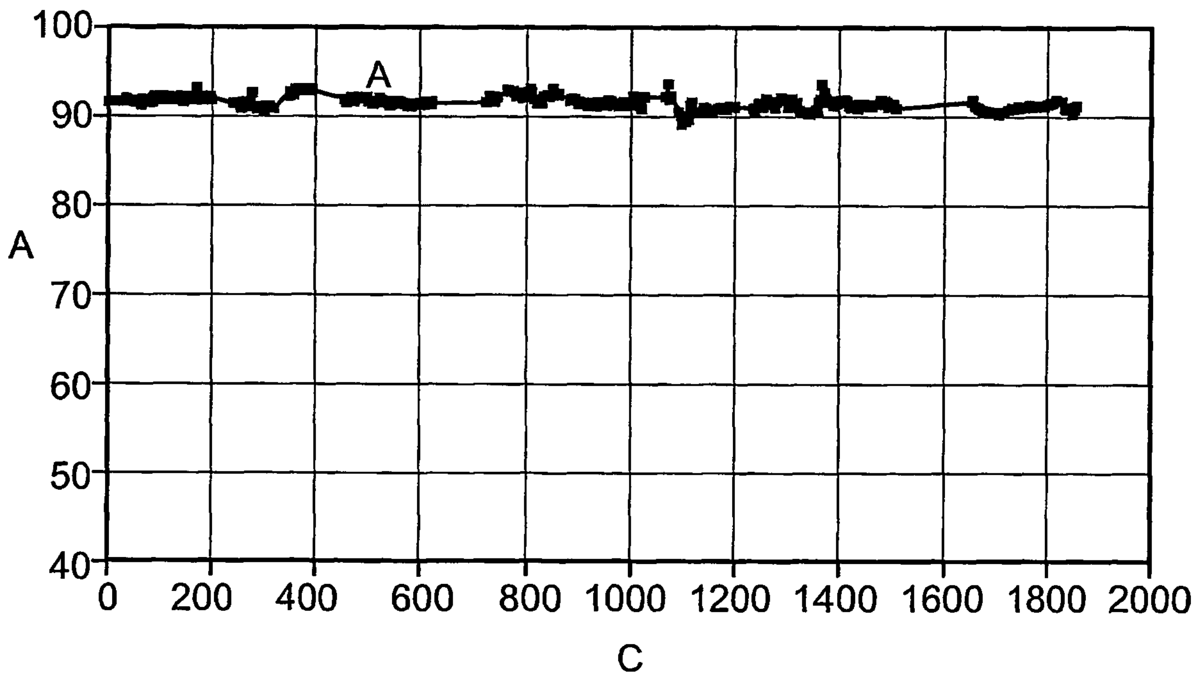
100
90
80
70
60
50
40
A
A
0
200
400
600
800
1000
1200
1400
1600
1800
2000
C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 08027126 A
- DE 1209570 B
- US 5350849 A


### In der Beschreibung aufgeführte Nicht-Patentliteratur


- *Thianranqi Huagong,* vol. 26, 23-25
- *CHEMICAL ABSTRACTS,* 136:135396
- *Turk. J. Chem.,* 1999, vol. 23, 319-327